# EUROPEAN PATENT SPECIFICATION

(11) **EP 2 392 378 B1**
(45) Date of publication and mention of the grant of the patent: **15.03.2017**
(21) Application number: 10735768.3
(22) Date of filing: 25.01.2010
(51) Int. Cl.: A61K 38/18, A61K 38/19, A61K 38/21, A61K 38/24, A61K 38/26, A61L 27/00, A61K 9/00, A61K 47/10, A61K 47/32, A61M 37/00

(54) **MICRONEEDLE DEVICE**
MIKRONADELVORRICHTUNG
DISPOSITIF DE MICRO-AIGUILLE

(30) Priority: 30.01.2009 JP 2009020672
(43) Date of publication of application: 07.12.2011
(73) Proprietor: Hisamitsu Pharmaceutical Co., Inc., Tosu-shi, Saga 841-0017 (JP)
(72) Inventor: KUWAHARA Tetsuji, Tsukuba-shi Ibaraki 305-0856 (JP); TOKUMOTO Seiji, Tsukuba-shi Ibaraki 305-0856 (JP); MATSUDO Toshiyuki, Tsukuba-shi Ibaraki 305-0856 (JP)
(74) Representative: Grünecker Patent- und Rechtsanwälte PartG mbB
(86) International application number: PCT/JP2010/050892
(87) International publication number: WO 2010/087300

(56) References cited:
- EP-A1- 1 992 386
- EP-A1- 2 119 469
- EP-A1- 2 153 863
- WO-A1-2007/091608
- WO-A1-2008/139648
- JP-T- 2008 520 370
- US-A1- 2006 034 903

## Description

### Technical Field

The present invention relates to a microneedle device having a plurality of microneedles capable of piercing the skin on a base for administration of drugs through the skin.

### Background Art

Conventionally, a microneedle device has been known as a device for improving transdermal absorption of drugs. Microneedles disposed on a microneedle device are intended to pierce the stratum corneum, the outermost skin layer, and various sizes and shapes have been proposed. A microneedle device is desired as a non-invasive administration method (refer to Patent Literature 1).

Further, various methods have also been proposed with regard to a method of applying drugs in which a microneedle device is used. In Patent Literature 2, coating the surface of microneedles with drugs, forming a groove or a hollow part in microneedles through which drugs or body components are allowed to penetrate, mixing drugs into microneedles themselves, and the like are described. Further, in Patent Literature 2, it is also stated that it is preferable that a reservoir medium contain sugars, particularly, sugars for stabilization such as lactose, raffinose, trehalose, or sucrose, which forms glass (noncrystalline solid material).

Patent Literature 3 discloses an apparatus for percutaneous administration of an influenza vaccine. Patent Literature 3 describes examples of a coating formulation applied to a microprojection array, and the examples include poly(vinyl alcohol), poly(ethylene oxide), poly(2-hydroxyethyl methacrylate), poly(n-vinylpyrrolidone), and polyethylene glycol. Also, Patent Literature 3 states that the desired coating thickness depends on several factors such as the coating thickness necessary to administer the required dose, the density of the microprojections per unit area of the sheet, the viscosity and composition of the coating formulation, as well as the coating method to be chosen.

US 2006/034903 relates to a microneedle device for delivering a protein, the device having a coating comprising the protein present in an amount ranging from 1-30 wt.% in the formulation and a water-soluble polymer such as PEG or PVP in an amount ranging from 0.03-10 wt. %. EP 2 119 469 discloses a microneedle device with a coating including a protein and a water-soluble polymer such as PEG, which is specially adapted for high molecular weight active agents. The amount of active agent is ranging from and the amount of polymer is ranging from 3 to 25% by weight. The concentration of the active agent in the liquid coating solution or suspension can be in the range of 0.1 to 65% by weight, preferably in the range of 1 to 30% by weight, and more preferably 3 to 20% by weight. EP 2 153 863 discloses a coating for a microneedle device comprising a protein such as BSA in an amount of 3 to 20% by weight and a water-soluble polymer such as PEG or PVP in an amount of 3 to 25% by weight.

### Citation List

### Patent Literature

Patent Literature 1: National Publication of International Patent Application No. 2001-506904
Patent Literature 2: National Publication of International Patent Application No. 2004-504120
Patent Literature 3: National Publication of International Patent Application No. 2007-530680

### Summary of Invention

### Technical Problem

By the way, it is reported that in order to coat a needle tip of a microneedle with a desired amount of physiologically active ingredients (low molecular weight compounds and high molecular weight ingredients such as peptide and protein), it is effective to add a carrier (a thickening agent) in a coating liquid. Actually, it is reported that a water-soluble polymer such as PVA enables an efficient drug delivery (WO2007-091608).

However, in the case where the physiologically active ingredients are limited to high molecular weight physiologically active ingredients such as peptide and protein, mixing of most of water-soluble polymers to be used as a carrier with high molecular weight active ingredients (peptide, protein, and the like) causes an aggregation phenomenon or a phase separation phenomenon, which makes it difficult to obtain a uniform coating liquid. In order to solve this problem, obtaining a uniform coating liquid by adding a surfactant can be considered. However, some physiologically active ingredients are unstable, which may pose a new problem that activity is eliminated by a surfactant.

If a coating liquid does not uniformly contain high molecular weight active ingredients, highly precise control of the amount of coating of microneedles with high molecular weight active ingredients cannot be carried out, and furthermore, coating itself may be difficult. Therefore, it is important to obtain a dissolved coating agent uniformly containing high molecular weight active ingredients.

The present invention has been made to solve the above-mentioned problems, and has an object to provide a microneedle device including a coating agent that contains high molecular weight active ingredients substantially uniformly.

### Solution to Problem

In order to solve the above-mentioned problems, the present inventors have keenly studied and carried out screening of coating carriers. As a result, they have found that the use of some water-soluble polymers enables reliable uniform mixing of high molecular weight active ingredients without causing an aggregation phenomenon or a phase separation phenomenon. Furthermore, the present inventors have also found that setting the concentration of the water-soluble polymers at 0.1 to 30 wt% enables efficient coating with the high molecular weight active ingredients, and have reached the present invention.

That is to say, the microneedle device of the present invention includes a base, and microneedles capable of piercing a skin and being disposed on the base, wherein at least a part of a surface of the microneedles and/or the base is coated with a coating agent containing a high molecular weight active ingredient and a water-soluble polymer, a content of the water-soluble polymer in the coating agent is 0.1 to 30% by weight, and the ratio of the content of the water-soluble polymer to a content of the high molecular weight active ingredient in the coating agent is 5:1 to 1:100.

In such a microneedle device, since the coating agent contains a water-soluble polymer that is compatible with high molecular weight active ingredients, the high molecular weight active ingredients can be allowed to be contained in the coating agent substantially uniformly. As a result, it is possible to coat microneedles with high molecular weight active ingredients with a high concentration while suppressing aggregation and phase separation of the high molecular weight active ingredients. Furthermore, by setting the content of the water-soluble polymer in the coating agent at 0.1 to 30% by weight, coating with the high molecular weight active ingredients can be conducted efficiently, and the usability of the microneedle device can be specifically improved.

In the microneedle device of the present invention, the water-soluble polymer is a carboxyvinyl polymer, and the content of the water-soluble polymer in the coating agent may be 0.5 to 10% by weight.

In the microneedle device of the present invention, the water-soluble polymer is a carboxyvinyl polymer, and the ratio of the content of the water-soluble polymer to the content of the high molecular weight active ingredient in the coating agent may be 1:3 to 1:80.

Thus, by adjusting the amounts of the water-soluble polymer and the high molecular weight active ingredients, the amount of the coating can be precisely controlled so as to specifically improve the convenience of the microneedle device.

### Advantageous Effects of Invention

According to such a microneedle device, because a coating agent contains a water-soluble polymer compatible with high molecular weight active ingredients, it is possible to allow the high molecular weight active ingredients to be contained in the coating agent substantially uniformly.

### Brief Description of Drawings

[Figure 1] Figure 1 is a perspective view showing one example of the microneedle device according to an embodiment.
[Figure 2] Figure 2 is a cross sectional view taken along the line II-II in Figure 1.
[Figure 3] Notations (a) to (c) are diagrams showing one example of a coating method of the microneedles.

### Description of Embodiments

Hereinbelow, the embodiments of the present invention will be described in detail with reference to attached drawings. It is to be noted that identical signs are assigned to identical or equivalent elements and redundant description is omitted in the description of the drawings.

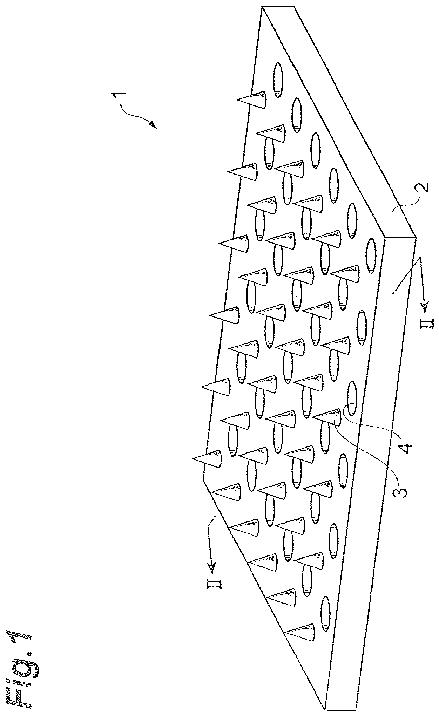

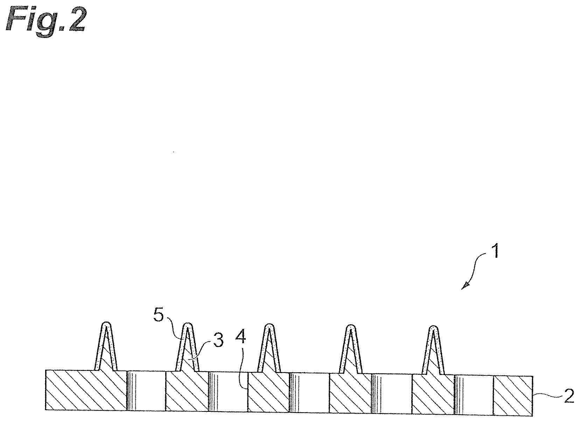

Figure 1 is a perspective view showing one example of the microneedle device according to an embodiment. Figure 2 is a cross sectional view taken along the line II-II in Figure 1.

As shown in Figure 1, a microneedle device 1 has a microneedle base 2 and a plurality of microneedles 3 capable of piercing the skin two-dimensionally arranged on the microneedle base 2.

The microneedle base 2 is a foundation to support the microneedles 3. On the microneedle base 2, a plurality of through-holes 4 are formed so that they are two-dimensionally arranged. The microneedles 3 and the through-holes 4 are alternately arranged in the direction of a diagonal of the microneedle base 2. By through-holes 4, it becomes possible to administer physiologically active ingredients from the back of the microneedle base 2. However, a base lacking such a through-hole may also be used. The area of the microneedle base 2 is 0.5 cm² to 10 cm², preferably 1 cm² to 5 cm², and more preferably 1 cm² to 3 cm². It may also be possible to configure a base of a desired size by connecting several of these microneedle bases 2.

The microneedles 3 each have a minute structure, and a height (length) thereof h is preferably 50 to 600 µm. At this point, the reason for setting the length of the microneedles 3 at 50 µm or more is to ensure transdermal administration of active ingredients, and the reason for setting the length at 600 µm or less is to avoid the contact between the microneedles and nerves so as to securely reduce the possibility of pain and securely avoid the possibility of bleeding. Also, when the length of the microneedles 3 is 500 µm or less, the amount of active ingredients to be released inside the skin can be efficiently administered. It is particularly preferable that the length of the microneedles 3 be 300 to 500 µm.

At this point, a microneedle refers to a projecting structure including, in a broad sense, a needle shape or a structure containing a needle shape. However, the microneedle is not limited to a structure having a needle shape with a tapered tip but also includes a structure lacking a tapered tip. When the microneedles 3 are in a conical shape, a diameter of the basal surface thereof is approximately 50 to 200 µm. Although the microneedles 3 are in a conical shape in the present embodiment, microneedles in a polygonal pyramid shape such as a square pyramid and the like may also be used.

As to a density of the microneedles 3, the microneedles are typically spaced apart so that a density of approximately one to 10 needles per millimeter (mm) is provided in a row of the needles. Generally, adjacent rows are spaced apart from each other by a distance substantially equal to the space between the needles in a row, and the needle density is 100 to 10000 needles per cm². When there is a needle density of 100 needles or more, the needles can efficiently pierce the skin. Meanwhile, a needle density of more than 10000 needles makes it difficult to give the microneedles 3 strength capable of piercing the skin. The density of the microneedles 3 is preferably 200 to 5000 needles, more preferably 300 to 2000 needles, and most preferably 400 to 850 needles.

While examples of a material of the microneedle base 2 or the microneedles 3 include silicon, silicon dioxide, ceramics, metals (such as stainless steel, titanium, nickel, molybdenum, chromium, and cobalt), and synthetic or natural resin materials, in consideration of the antigenicity of the microneedle and the unit price of the material, a biodegradable polymer such as polylactic acid, polyglycolide, polylactic acid-CO-polyglycolide, pullulan, capronolactone, polyurethane, and polyanhydride, and a synthetic or natural resin material such as polycarbonate, polymethacrylic acid, ethylenevinyl acetate, polytetrafluoroethylene, and polyoxymethylene, which are non-biodegradable polymers, are particularly preferable. Further, polysaccharide such as hyaluronic acid, sodium hyaluronate, pullulan, dextran, dextrin, or chondroitin sulfate is also suitable.

Examples of a production method of the microneedle base 2 or the microneedles 3 include wet etching process or dry etching process using a silicon base, precision machining using metals or resins (such as electric discharge method, laser processing, dicing processing, hot embossing process, and injection mold processing), and machinery cutting. By these processing methods, a needle part and a support part are molded into an integrated unit. Examples of a method for hollowing the needle part include a method in which, following the production of the needle part, a secondary processing such as laser processing is performed.

On the microneedles 3, a coating 5 of a coating agent containing high molecular weight active ingredients and water-soluble polymer is provided. The coating 5 is a coating in which a coating liquid containing high molecular weight active ingredients and compatible water-soluble polymer is fixed to a part or all of the microneedles 3 and/or the microneedle base 2. Herein, the term "high molecular weight active ingredient" refers to a physiologically active substance having a molecular weight of 1000 or more. The term "compatible with" refers to being in a state, in a range of a visual evaluation, in which no phase separation and no formation of aggregation are observed in the centrifugation after a solution is prepared. Examples of the water-soluble polymer compatible with the high molecular weight active ingredients include the below-mentioned carboxyvinyl polymer, polyethylene oxide, and polyvinyl pyrrolidone. The term "fixed" refers to a state in which a coating liquid remains attached to an object almost uniformly. Immediately after coating, a coating liquid is fixed in a dried state by a known drying method, namely air drying, vacuum drying, freeze drying, or a combination thereof. However, after transdermal administration, the coating does not necessarily remain fixed in a dried state because it may contain a water content that is in equilibrium with the surrounding atmosphere or an organic solvent, or the like.

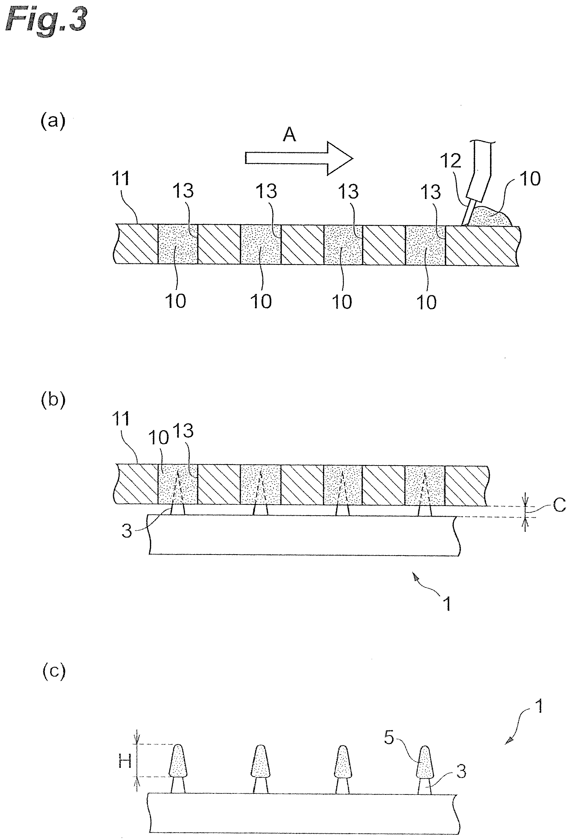

Figure 3 (a) to (c) are diagrams showing one example of a coating method of the microneedles 3. According to this method, firstly, as shown in Figure 3 (a), a coating liquid 10 is swept in the direction of an arrow A by a squeegee 12 on a mask plate 11 so as to fill openings 13 with the coating liquid 10. Subsequently, as shown in Figure 3 (b), the microneedles 3 are dipped into the openings 13 of the mask plate 11. Thereafter, as shown in Figure 3 (c), the microneedles 3 are pulled out of the openings 13 of the mask plate 11. By the above operation, the coating 5 of the coating liquid 10 is provided on the microneedles 3. The coating 5 is fixed to the microneedles 3 by drying.

A range of coating H of the microneedles 3 is controlled by clearance (gap) C shown in Figure 3 (b). This clearance C is defined as a distance between the basal surface of the microneedles 3 and the bottom surface of the mask plate 11 (base thickness is not involved), and is set according to a tension of the mask plate 11 and the length of the microneedles 3. A range of the distance of clearance C is preferably 0 to 500 µm. When the distance of clearance C is 0, the whole of the microneedles 3 is coated. Although the range of coating H varies depending on the height of the microneedles 3 h, it may be set at 0 to 500 µm, and it is normally 10 to 500 µm, and preferably approximately 30 to 300 µm.

A thickness of the coating 5 of the microneedles 3 is less than 50 µm, preferably less than 25 µm, and more preferably 1 to 10 µm. Generally, the thickness of coating is an average thickness as measured over the surface of the microneedles 3 after drying. The thickness of coating can generally be increased by applying multiple films of the coating carrier, namely, by repeating a coating process after fixation of the coating carrier.

When coating is performed on the microneedles 3, in order to minimize changes in drug concentrations and physical properties caused by volatilization of a solvent of the coating agent, it is preferable to control temperature and humidity in an installation environment of an apparatus at a constant level. In order to prevent solvent evaporation, it is preferable to either decrease the temperature or increase the humidity, or control both. The humidity at room temperature when the temperature is not controlled is, as a relative humidity, 50 to 100% RH, preferably 70 to 100% RH, and most preferably 90 to 100% RH. When the humidity is 50% RH or less, significant solvent evaporation occurs, causing physical properties of a coating solution to change. Although a humidification method includes a vapor system, a steam vapor system, a water spray system, and the like, no particular limitation is imposed thereon as long as an intended humidity condition is assured. As a thickening agent mixed into the coating solution, it is preferable to select a water-soluble polymer, which has high wettability and moisture retaining properties that minimize the volatility of the solvent.

The coating agent contains physiologically active ingredients and purified water and/or high molecular weight coating carriers. Examples of the high molecular weight coating carrier include polyethylene oxide, polyhydroxymethylcellulose, hydroxypropylcellulose, polyhydroxypropylmethylcellulose, polymethylcellulose, dextran, polyethylene glycol, polyvinyl alcohol, polyvinyl pyrrolidone, pullulan, carmellose sodium, chondroitin sulfate, hyaluronic acid, sodium hyaluronate, dextrin, and gum arabic.

As the coating carrier, a water-soluble high molecular weight carrier that is compatible (having the property of being homogeneously mixed) with high molecular weight active ingredients is preferable. Specifically, polyvinyl pyrrolidone, polyvinyl alcohol, carboxyvinyl polymer, polyacrylic acid, sodium polyacrylate, polyoxyethylene polyoxypropylene glycol, Pluronic, polyethylene oxide, polyethylene glycol, polyvinyl acetamide, and the like, are preferable. Carboxyvinyl polymer, polyethylene oxide, and polyvinyl pyrrolidone are particularly preferable. Carboxyvinyl polymer is most preferable.

A content of the coating carrier in the coating agent is 0.1 to 70% by weight, preferably 0.1 to 60% by weight, and particularly preferably 0.1 to 30% by weight. The coating carrier may have to have a certain degree of viscosity so as not to drip, and a viscosity of approximately 100 to 100000 cps is necessary. A more preferable viscosity is 500 to 60000 cps. For the viscosity being within the above range, it becomes possible to apply a desired amount of a coating solution at once without depending on the material of the microneedles 3. Also, generally, there is a tendency that the higher the viscosity, the larger the amount of a coating solution.

A content of carboxyvinyl polymer in the coating agent is preferably 0.5 to 10% by weight, and particularly preferably about 1% by weight.

A liquid composition used for coating the microneedles 3 is prepared by mixing biocompatible carriers, beneficial physiologically active ingredients to be delivered, and in some cases, any of coating aids with a volatile liquid. The volatile liquid can be water, dimethyl sulfoxide, dimethyl formamide, ethanol, isopropyl alcohol, and a mixture thereof. Among them, water is most preferable. A coating solution in a liquid state or a suspension can typically have a concentration of beneficial physiologically active ingredients of 0.1 to 65% by weight, preferably 1 to 40% by weight, more preferably 10 to 30% by weight. The coating is particularly preferably in a fixed state. A surfactant can be zwitter ion type, amphoteric ion type, cationic type, anionic type, or nonionic type. For example, the surfactant can be Tween 20 and Tween 80, other sorbitan derivatives such as sorbitan laurate, and alkoxylated alcohols such as Laureth-4. For example, in order to dissolve a larger amount of high molecular weight active ingredients into the coating carrier, adding surfactants is also effective.

Other known pharmaceutical additives may be added to the coating as long as they do not adversely affect a necessary solubility and characteristics of the viscosity of the coating as well as nature and physical properties of the dried coating.

The high molecular weight active ingredients (drugs) used in the present invention are high molecular weight compounds. High molecular weight refers to, as an index, a molecular weight of 1000 or more, and an upper limit of the molecular weight is not particularly set. As the high molecular weight compound, peptide, protein, DNA, RNA, and the like, are considered, but no particular limitation is imposed. Examples include α-interferon, β-interferon for multiple sclerosis, erythropoietin, follicle stimulating hormone (FSH), follitropin β, follitropin α, G-CSF, GM-CSF, human chorionic gonadotropin, leutinizing hormone, salmon calcitonin, glucagon, GNRH antagonist, insulin, human growth hormone, filgrastim, heparin, low molecular weight heparin, parathyroid hormone (PTH), and somatropin. Also, examples of a vaccine include influenza vaccine, Japanese encephalitis vaccine, rotavirus vaccine, Alzheimer's disease vaccine, arteriosclerosis vaccine, cancer vaccine, nicotine vaccine, diphtheria vaccine, tetanus vaccine, pertussis vaccine, Lyme disease vaccine, rabies vaccine, diplococcus pneumoniae vaccine, yellow fever vaccine, cholera vaccine, vaccinia vaccine, tuberculosis vaccine, rubella vaccine, measles vaccine, mumps vaccine, botulism vaccine, herpes vaccine, other DNA vaccines, and hepatitis B vaccine.

Furthermore, physiologically active substances may be vaccine, low molecular weight peptide, saccharide, nucleic acid, and the like, as long as they have a molecular weight of about 1000.

It is to be noted that these drugs may be used singly or two or more kinds thereof may be used in combination. As long as the salt is pharmaceutically acceptable, needless to say, a drug in a form of either an inorganic salt or an organic salt is encompassed.

In order to coat the microneedles 3 with high molecular weight active ingredients (drugs) used in the present invention reliably and efficiently, the rate of the high molecular weight active ingredients (drugs) and the amount of the coating carrier is particularly important. This is because the high molecular weight active ingredients (drugs) and the coating carrier are required to be mixed with each other uniformly with high compatibility, and to be deposited on the microneedles with high viscosity.

The ratio of the content of the coating carrier to the content of the high molecular weight active ingredients (drugs) is preferably 5:1 to 1:100, and further preferably 2:1 to 1:80. The ratio of the content of carboxyvinyl polymer to the content of the high molecular weight active ingredients (drugs) is preferably 1:3 to 1:80. In this way, by setting the ratio of the content at a preferable ratio of the content according to the properties of the respective polymers, a coating agent contains a high dose of high molecular weight active ingredients (drugs), and thus the coating agent can be preferably deposited on the microneedles 3 without dripping. Furthermore, the coating agent can be highly filled in a mask plate 11 (Figure 3), and since the coating agent is securely fixed to the microneedles 3 after dried following to coating, it is possible to prevent the coating agent from dropping during transport and/or operation of piercing a skin.

When administration is performed using the microneedle device 1, an auxiliary device for fixing the microneedle device 1 may be used, or direct administration by hand-pushing may be possible. When the microneedle device 1 is contacted with the skin, it is administered by a force of 1.0 to 10 kg, preferably by a force of 1.0 to 7 kg, and more preferably by a force of 1.0 to 4 kg. Also, administration time at such a force is not so long, and it is from several seconds to several minutes at longest, and depending on the case, instant administration that takes less than a second is also possible. However, it is also possible to fix the microneedle device 1 on the skin thereafter for continuous administration of active ingredients.

### Examples

Hereinbelow, the present invention will be specifically described based on Examples; however, the present invention is not limited in any way to these Examples.

### (Example 1) Test to Confirm Compatibility between Various Polymers and BSA and OVA

### (Operation Procedure)

As models of high molecular weight active ingredients, BSA (bovine serum albumin), OVA (ovalbumin) and TI (trypsin inhibitor) were employed, and mixed aqueous solutions of various polymers and BSA, OVA or TI were prepared according to the conditions shown in Tables 1 to 7 mentioned below. Then, by confirming presence or absence of formation of aggregate or presence or absence of the formation of phase separation after defoaming by centrifugation (centrifugation conditions are described in Tables), compatibility was evaluated (uniform liquid property = "o" and nonuniform liquid property = "x"). In Tables 1 to 7, "o" denotes that compatibility was observed, and "x" denotes that compatibility was not observed. It is to be noted that "%" in the following description denotes "% by weight" in the coating agent. After coating was carried out by the method shown in Figure 3, extraction was carried out with 1 mL of purified water, the content (deposition amount) of BSA, OVA or TI was determined by the BCA method and simultaneously the pH at that time was determined. The term "no coating" in the table shows that no deposition of polymers to the needles was observed.

The following various polymers were used. Polyvinyl pyrrolidone manufactured by Nippon Shokubai Co., Ltd., polyvinyl alcohol manufactured by Kuraray Co. Ltd., carboxyvinyl polymer manufactured by Nikko Chemicals Co., Ltd., polyacrylic acid (AC-10LP, AC-10LHP, and AC-10SHP) manufactured by Nihon Junyaku Co. Ltd., polyoxyethylene polyoxypropylene glycol manufactured by NOF Corporation, Pluronic manufactured by NOF Corporation, and sodium polyacrylate (NP-600 and NP-800) manufactured by Showa Denko K.K. were individually used. As polyethylene oxides, a grade having a molecular weight of 900000 manufactured by Union Carbide, and grades having a molecular weight of 2000000 and 5000000 manufactured by SIGMA, were individually used. Furthermore, polyethylene glycol manufactured by NOF Corporation, and vinyl acetamide manufactured by Nippon Carbide Industries Co., Inc. were individually used. It is to be noted that the approximate viscosity (mPa·s/20°C·0.2%) of the carboxyvinyl polymer manufactured by Nikko Chemicals Co., Ltd. is 16000 to 28000 for grade 980, 1500 to 7500 for grade 981, and 2000 to 12000 for grade ETD2050.

Table 1 shows the results with respect to the compatibility between OVA or BSA and various water-soluble polymers. Tables 2 and 3 show the compatibility between OVA or BSA and carboxyvinyl polymer, and the content of OVA or BSA when microneedles were coated. Tables 4 and 5 show the compatibility between OVA or BSA and polyethylene oxide, and the content of OVA or BSA when microneedles were coated. Tables 6 and 7 show the compatibility between BSA or TI and polyvinyl pyrrolidone, and the content of BSA or TI when microneedles were coated.

**[Table 1]**

| (Compatibility of Various Polymers with OVA or BSA) | | | | | | | |
|---|---|---|---|---|---|---|---|
| Polymer | Polymer (%) | OVA (%) | Compatibility | | Polymer (%) | BSA (%) | Compatibility |
| Polyvinyl pyrrolidone (average molecular weight: 58,000) | 30 | 20 | × | | 35 | 20 | × |
| Polyvinyl pyrrolidone (average molecular weight: 58,000) | 40 | 13.3 | × | | 52.5 | 10 | × |
| Polyvinyl pyrrolidone (average molecular weight: 58,000) | 20 | 26.7 | × | | - | - | - |
| Polyvinyl pyrrolidone (average molecular weight: 1,300,000) | 15 | 20 | ○ | | 15 | 20 | × |
| Polyvinyl pyrrolidone (average molecular weight: 1,300,000) | 20 | 13.3 | ○ | | 22.5 | 10 | × |
| Polyvinyl pyrrolidone (average molecular weight: 1,300,000) | 10 | 26.7 | ○ | | 10 | 13.3 | × |
| Polyvinyl pyrrolidone (average molecular weight: 1,300,000) | 10 | 33 | ○ | | 24 | 8 | × |
| Polyvinyl pyrrolidone (average molecular weight: 1,300,000) | 10 | 40 | ○ | | 10 | 26.7 | × |
| Polyvinyl pyrrolidone (average molecular weight: 1,300,000) | - | - | - | | 6 | 32 | × |
| Polyvinyl alcohol 117 | 10 | 16.7 | × | | 10 | 16.7 | × |
| Polyvinyl alcohol 117 | 5 | 16.7 | × | | 5 | 16.7 | × |
| Polyvinyl alcohol 220 | 10 | 20 | × | | 10 | 20 | × |
| Polyvinyl alcohol 220 | 13.3 | 13.3 | × | | 13.3 | 13.3 | × |
| Polyvinyl alcohol 220 | 6.7 | 26.7 | × | | 6.7 | 26.7 | × |
| Carboxyvinyl polymer 980 | 1.5 | 20 | ○ | | 1.5 | 20 | × |
| Carboxyvinyl polymer 980 | 2 | 13.3 | ○ | | 2 | 13.3 | × |
| Carboxyvinyl polymer 980 | 1 | 26.7 | ○ | | 1 | 26.7 | ○ |
| Carboxyvinyl polymer 980 | 1 | 33 | ○ | | 1 | 33 | ○ |
| Carboxyvinyl polymer 980 | 0.5 | 33 | × | | 1 | 40 | ○ |
| Carboxyvinyl polymer 980 | 0.3 | 36 | × | | 0.5 | 33 | × |
| Carboxyvinyl polymer 980 | - | - | - | | 0.3 | 36 | × |
| Carboxyvinyl polymer 981 | 5 | 20 | × | | 5 | 20 | × |
| Carboxyvinyl polymer 981 | 6.7 | 13.3 | × | | 6.7 | 13.3 | × |
| Carboxyvinyl polymer 981 | 3.3 | 26.7 | ○ | | 3.3 | 26.7 | × |
| Carboxyvinyl polymer 981 | 1 | 36 | ○ | | 1 | 36 | ○ |
| Carboxyvinyl polymer 981 | 0.5 | 36 | × | | 0.5 | 36 | × |
| Carboxyvinyl polymer (ETD2050) | 5 | 20 | × | | 5 | 20 | × |
| Carboxyvinyl polymer (ETD2050) | 6.7 | 13.3 | ○ | | 6.7 | 13.3 | × |
| Carboxyvinyl polymer (ETD2050) | 3.3 | 26.7 | ○ | | 3.3 | 26.7 | × |
| Carboxyvinyl polymer (ETD2050) | 1 | 36 | ○ | | 1 | 36 | ○ |
| Carboxyvinyl polymer (ETD2050) | 0.5 | 36 | × | | 0.5 | 36 | × |
| Polyacrylic acid (AC-10LP) (molecular weight: 20000 to 30000) | 30 | 20 | ○ | | 30 | 20 | × |
| Polyacrylic acid (AC-10LP) (molecular weight: 20000 to 30000) | 40 | 13.3 | × | | 40 | 13.3 | × |
| Polyacrylic acid (AC-10LP) (molecular weight: 20000 to 30000) | 20 | 26.7 | × | | 20 | 26.7 | × |
| Polyacrylic acid (AC-10LHP) (molecular weight: 200000 to 300000) | 10 | 20 | × | | 10 | 20 | × |
| Polyacrylic acid (AC-10LHP) (molecular weight: 200000 to 300000) | 13.3 | 13.3 | × | | 13.3 | 13.3 | × |
| Polyacrylic acid (AC-10LHP) (molecular weight: 200000 to 300000) | 6.7 | 26.7 | × | | 6.7 | 26.7 | × |
| Polyacrylic acid (AC-10SHP) (molecular weight: 1000000 to 1500000) | 5 | 20 | × | | 5 | 20 | × |
| Polyacrylic acid (AC-10SHP) (molecular weight: 1000000 to 1500000) | 6.7 | 13.3 | × | | 6.7 | 13.3 | × |
| Polyacrylic acid (AC-10SHP) (molecular weight: 1000000 to 1500000) | 3.3 | 26.7 | ○ | | 3.3 | 26.7 | × |
| Polyoxyethylene polyoxypropylene glycol (200E.O.X70P.O) | 20 | 20 | × | | 20 | 20 | × |
| Polyoxyethylene polyoxypropylene glycol (200E.O.X70P.O) | 26.7 | 13.3 | × | | 26.7 | 13.3 | ○ |
| Polyoxyethylene polyoxypropylene glycol (200E.O.X70P.O) | 13.3 | 26.7 | × | | 13.3 | 26.7 | × |
| Pluronic F68 | 25 | 20 | × | | 25 | 20 | × |
| Pluronic F68 | 33.3 | 13.3 | × | | 33.3 | 13.3 | × |
| Pluronic F68 | 16.7 | 26.7 | × | | 16.7 | 26.7 | × |
| Sodium polyacrylate (NP-600) | 3 | 16.7 | ○ | | 3 | 16.7 | ○ |
| Sodium polyacrylate (NP-600) | 1.5 | 16.7 | ○ | | 1.5 | 16.7 | ○ |
| Sodium polyacrylate (NP-800) | 3 | 16.7 | ○ | | 3 | 16.7 | ○ |
| Sodium polyacrylate (NP-800) | 1.5 | 16.7 | ○ | | 1.5 | 16.7 | ○ |
| Polyethylene oxide (MW900000) | 3 | 16.7 | ○ | | 3 | 16.7 | ○ |
| Polyethylene oxide (MW900000) | 3 | 30 | ○ | | 3 | 30 | × |
| Polyethylene oxide (MW900000) | 3 | 40 | × | | 1.5 | 16.7 | ○ |
| Polyethylene oxide (MW900000) | 1.5 | 16.7 | ○ | | 1.5 | 30 | × |
| Polyethylene oxide (MW900000) | 1.5 | 30 | ○ | | 1.5 | 40 | × |
| Polyethylene oxide (MW900000) | 1.5 | 40 | × | | - | - | - |
| Polyethylene oxide (MW2000000) | 2 | 20 | ○ | | 2 | 20 | × |
| Polyethylene oxide (MW2000000) | 2.7 | 13.3 | ○ | | 2.7 | 13.3 | ○ |
| Polyethylene oxide (MW2000000) | 1.3 | 26.7 | × | | 1.3 | 26.7 | × |
| Polyethylene oxide (MW5000000) | 1.5 | 20 | ○ | | 1.5 | 20 | ○ |
| Polyethylene oxide (MW5000000) | 2 | 13.3 | ○ | | 2 | 13.3 | ○ |
| Polyethylene oxide (MW5000000) | 1 | 26.7 | × | | 1 | 26.7 | × |
| Polyethylene glycol 20000 | 20 | 16.7 | × | | 20 | 16.7 | × |
| Polyethylene glycol 20000 | 10 | 16.7 | × | | 10 | 16.7 | × |
| Polyvinyl acetamide | 3 | 16.7 | × | | - | - | - |
| Polyvinyl acetamide | 1.5 | 16.7 | ○ | | - | - | - |

**[Table 2]**

| (Compatibility of Carboxyvinyl Polymer with OVA and Content in Coating) | | | | | |
|---|---|---|---|---|---|
| Polymer | Polymer (%) | OVA (%) | Compatibility (centrifugation condition) | pH | Content in coating (µg) |
| Carboxyvinyl polymer 980 | 1.5 | 20 | ○ (15000rpm × 2min) | - | 18 |
| Carboxyvinyl polymer 980 | 2 | 13.3 | ○ (15000rpm × 2min) | - | 10 |
| Carboxyvinyl polymer 980 | 1 | 26.7 | ○ (15000rpm × 2min) | - | 23 |
| Carboxyvinyl polymer 980 | 1 | 33 | ○ (15000rpm × 2min) | 5.4 | 55 |
| Carboxyvinyl polymer 980 | 0.5 | 33 | × | - | - |
| Carboxyvinyl polymer 980 | 0.3 | 36 | × | - | - |
| Carboxyvinyl polymer 981 | 5 | 20 | × | - | - |
| Carboxyvinyl polymer 981 | 6.7 | 13.3 | × | - | - |
| Carboxyvinyl polymer 981 | 3.3 | 26.7 | ○ (15000rpm × 15min) | - | No coating |
| Carboxyvinyl polymer 981 | 1 | 36 | ○ (15000rpm × 2min) | 5.2 | 25 |
| Carboxyvinyl polymer 981 | 0.5 | 36 | × | - | - |
| Carboxyvinyl polymer (ETD2050) | 5 | 20 | × | - | - |
| Carboxyvinyl polymer (ETD2050) | 6.7 | 13.3 | ○ (15000rpm × 10min) | 3.8 | 10 |
| Carboxyvinyl polymer (ETD2050) | 3.3 | 26.7 | ○ (15000rpm × 2min) | 4.6 | 21 |
| Carboxyvinyl polymer (ETD2050) | 1 | 36 | ○ (15000rpm × 2min) | 5.3 | 89 |
| Carboxyvinyl polymer (ETD2050) | 0.5 | 36 | × | - | - |

**[Table 3]**

| (Compatibility of Carboxyvinyl Polymer with BSA and Content in Coating) | | | | | |
|---|---|---|---|---|---|
| Polymer | Polymer (%) | BSA (%) | Compatibility (centrifugation condition) | pH | Content in coating (µg) |
| Carboxyvinyl polymer 980 | 1.5 | 20 | × | - | - |
| Carboxyvinyl polymer 980 | 2 | 13.3 | × | - | - |
| Carboxyvinyl polymer 980 | 1 | 26.7 | ○ (15000rpm × 2min) | - | 21 |
| Carboxyvinyl polymer 980 | 1 | 33 | ○ (15000rpm × 2min) | 5.4 | 135 |
| Carboxyvinyl polymer 980 | 1 | 40 | ○ (15000rpm × 2min) | 5.5 | 129 |
| Carboxyvinyl polymer 980 | 0.5 | 33 | ○ | - | - |
| Carboxyvinyl polymer 980 | 0.3 | 36 | × | - | - |
| Carboxyvinyl polymer 981 | 5 | 20 | × | - | - |
| Carboxyvinyl polymer 981 | 6.7 | 13.3 | × | - | - |
| Carboxyvinyl polymer 981 | 3.3 | 26.7 | × | - | - |
| Carboxyvinyl polymer 981 | 1 | 36 | ○ (15000rpm × 2min) | 5.4 | 93 |
| Carboxyvinyl polymer 981 | 0.5 | 36 | × | - | - |
| Carboxyvinyl polymer (ETD2050) | 5 | 20 | × | - | - |
| Carboxyvinyl polymer (ETD2050) | 6.7 | 13.3 | × | - | - |
| Carboxyvinyl polymer (ETD2050) | 3.3 | 26.7 | × | - | - |
| Carboxyvinyl polymer (ETD2050) | 1 | 36 | ○ (15000rpm × 2min) | 5.4 | 123 |
| Carboxyvinyl polymer (ETD2050) | 0.5 | 36 | × | - | - |

**[Table 4]**

| Compatibility of Polyethylene Oxide Polymer with OVA and Content in Coating) | | | | | |
|---|---|---|---|---|---|
| Polymer | Polymer (%) | OVA (%) | Compatibility (centrifugation condition) | pH | Content in coating (µg) |
| Polyethylene oxide (MW900000) | 3 | 16.7 | ○ (15000rpm × 2min) | - | 7 |
| Polyethylene oxide (MW900000) | 3 | 30 | ○ (15000rpm × 2min) | - | 11 |
| Polyethylene oxide (MW900000) | 3 | 40 | × | - | - |
| Polyethylene oxide (MW900000) | 1.5 | 16.7 | ○ (15000rpm × 2min) | - | 4 |
| Polyethylene oxide (MW900000) | 1.5 | 30 | ○ (15000rpm × 2min) | - | 9 |
| Polyethylene oxide (MW900000) | 1.5 | 40 | × | - | - |
| Polyethylene oxide (MW2000000) | 2 | 20 | ○ (15000rpm × 2min) | 7.0 | 17 |
| Polyethylene oxide (MW2000000) | 2.7 | 13.3 | ○ (15000rpm × 2min) | 7.1 | 15 |
| Polyethylene oxide (MW2000000) | 1.3 | 26.7 | × | - | - |
| Polyethylene oxide (MW5000000) | 1.5 | 20 | ○ (15000rpm × 2min) | 7.0 | 17 |
| Polyethylene oxide (MW5000000) | 2 | 13.3 | ○ (15000rpm × 2min) | 7.1 | 13 |
| Polyethylene oxide (MW5000000) | 1 | 26.7 | × | - | - |

**[Table 5]**

| (Compatibility of Polyethylene Oxide Polymer with BSA and Content in Coating) | | | | | |
|---|---|---|---|---|---|
| Polymer | Polymer (%) | BSA (%) | Compatibility (centrifugation condition) | pH | Content in coating (µg) |
| Polyethylene oxide (MW900000) | 3 | 16.7 | ○ (15000rpm × 2min) | - | 4 |
| Polyethylene oxide (MW900000) | 3 | 30 | × | - | - |
| Polyethylene oxide (MW900000) | 1.5 | 16.7 | ○ (5000rpm × 5min) | - | 6 |
| Polyethylene oxide (MW900000) | 1.5 | 30 | × | - | - |
| Polyethylene oxide (MW900000) | 1.5 | 40 | × | - | - |
| Polyethylene oxide (MW2000000) | 2 | 20 | × | - | - |
| Polyethylene oxide (MW2000000) | 2.7 | 13.3 | ○ (15000rpm × 2min) | 7.0 | 13 |
| Polyethylene oxide (MW2000000) | 1.3 | 26.7 | × | - | - |
| Polyethylene oxide (MW5000000) | 1.5 | 20 | ○ (15000rpm × 2min) | 6.8 | 12 |
| Polyethylene oxide (MW5000000) | 2 | 13.3 | ○ (15000rpm × 2min) | 6.9 | 8 |
| Polyethylene oxide (MW5000000) | 1 | 26.7 | × | - | - |

**[Table 6]**

| (Compatibility of Polyvinyl Pyrrolidone Polymer with BSA and Content in Coating) | | | | | |
|---|---|---|---|---|---|
| Polymer | Polymer (%) | BSA (%) | Compatibility (centrifugation condition) | pH | Content in coating (µg) |
| Polyvinyl pyrrolidone (average molecular weight: 58,000) | 30 | 20 | × | - | - |
| Polyvinyl pyrrolidone (average molecular weight: 58,000) | 40 | 13.3 | × | - | - |
| Polyvinyl pyrrolidone (average molecular weight: 58,000) | 20 | 26.7 | × | - | - |
| Polyvinyl pyrrolidone (average molecular weight: 1,300,000) | 15 | 20 | ○ (15000rpm × 2min) | 6.7 | 24 |
| Polyvinyl pyrrolidone (average molecular weight: 1,300,000) | 20 | 13.3 | ○ (15000rpm × 2min) | 6.5 | 22 |
| Polyvinyl pyrrolidone (average molecular weight: 1,300,000) | 10 | 26.7 | ○ (15000rpm × 2min) | 6.8 | 28 |
| Polyvinyl pyrrolidone (average molecular weight: 1,300,000) | 10 | 33 | ○ (15000rpm × 2min) | 6.9 | 75 |
| Polyvinyl pyrrolidone (average molecular weight: 1,300,000) | 10 | 40 | ○ (15000rpm × 5min) | 7.0 | 22 |

**[Table 7]**

| (Compatibility of Polyvinyl Pyrrolidone Polymer with TI and Content in Coating) | | | | | |
|---|---|---|---|---|---|
| Polymer | Polymer (%) | TI (%) | Compatibility (centrifugation condition) | pH | Content in coating (µg) |
| Polyvinyl pyrrolidone (average molecular weight: 1,300,000) | 15 | 20 | × | - | - |
| Polyvinyl pyrrolidone (average molecular weight: 1,300,000) | 20 | 13.3 | ○ (15000rpm × 2min) | 6.6 | 38 |
| Polyvinyl pyrrolidone (average molecular weight: 1,300,000) | 10 | 26.7 | × | - | - |

As shown in Table 1, when the blending ratio of physiologically active ingredients and water-soluble polymer is optimized, high compatibility with both OVA and BSA is observed in carboxyvinyl polymer and polyethylene oxide, and high compatibility with only OVA is observed in polyvinyl pyrrolidone. However, polyvinyl pyrrolidone shows relatively preferable compatibility with OVA when the average molecular weight is not 58000 but 1300000.

As shown in Tables 2 and 3, carboxyvinyl polymer (981) has a viscosity of 1500 to 7500 (mPa·s/20°C·0.2%), which is lower than that of the other grades, and it has a compatibility but the content in the coating tends to be lower, suggesting that the viscosity of the water-soluble polymer is involved in the content of BSA or OVA in the coating. In particular, in other grades 980 and ETD2050, it has been revealed that coating with high concentration OVA or BSA is possible when the polymer concentration is about 1%. Furthermore, since effective application is possible at pH 5.2 to 5.4, it is considered that carboxyvinyl polymer is suitable for application of high molecular weight active ingredients which are stable and dissolved (or uniformly dispersed) around this pH.

As shown in Tables 4 and 5, polyethylene oxide was confirmed to have a compatibility with OVA or BSA in substantially all the grades (molecular weights: 900000, 2000000, and 5000000). It has been revealed that relatively preferable coating is possible when the polymer concentration is 2 to 3%. Furthermore, since application is possible around the neutrality of pH 6.8 to 7.1, it is considered that polyethylene oxide is suitable for application of high molecular weight active ingredients which are stable and dissolved (or uniformly dispersed) around this pH.

As shown in Tables 6 and 7, it has been confirmed that polyvinyl pyrrolidone has a compatibility with OVA or TI in the grade of an average molecular weight of 1300000. It has been revealed that coating is possible at the polymer concentration of 10 to 20%. Furthermore, since application is possible, around weak acidity to neutrality, pH 6.5 to 7.0, it is considered that polyvinyl pyrrolidone is suitable for application of high molecular weight active ingredients which are stable and dissolved (or uniformly dispersed) around this pH.

### Industrial Applicability

According to the present invention, it is possible to uniformly coat microneedles with high molecular weight active ingredients. Also, since a solution is uniform, a highly precise coating of the microneedles is possible. Furthermore, the amount of coating is controlled so as to specifically enhance the convenience of the microneedle device by adjusting the amount of water-soluble polymer, and the present invention has industrial applicability.

### Reference signs List

1···microneedle device, 2···microneedle base, 3···microneedle, 4···through-hole, and 5···coating

## Claims

1. A microneedle device comprising:
a base; and
microneedles capable of piercing the skin and being disposed on the base,
wherein at least part of the surface of the microneedles and/or the base is coated with a coating agent comprising an active ingredient with a molecular weight of 1000 or more and a carboxyvinyl polymer, and wherein the coating is obtainable by applying a coating solution having a pH of 5.2 to 5.4,
the content of the carboxyvinyl polymer in the coating agent being 0.1 to 30% by weight,
and the weight ratio of the content of the carboxyvinyl polymer to the content of the active ingredient in the coating agent being 5:1 to 1:100.

2. The microneedle device according to claim 1, wherein the content of the carboxyvinyl polymer in the coating agent is 0.5 to 10% by weight.

3. The microneedle device according to claim 1, wherein the weight ratio of the content of the carboxyvinyl polymer to the content of the active ingredient in the coating agent is 1:3 to 1:80.

## Patentansprüche

1. Mikronadelvorrichtung umfassend:
eine Basis; und
Mikronadeln, welche die Haut durchdringen können und auf der Basis angeordnet sind,
wobei wenigstens ein Teil der Oberfläche der Mikronadeln und/oder der Basis mit einem Beschichtungsmittel beschichtet ist, das einen Wirkstoff mit einem Molekulargewicht von 1000 oder mehr und ein Carboxyvinylpolymer umfasst, und wobei die Beschichtung durch Aufbringen einer Beschichtungslösung mit einem pH von 5,2 bis 5,4 erhältlich ist,
wobei der Gehalt des Carboxyvinylpolymers in dem Beschichtungsmittel 0,1 bis 30 Gew.-% beträgt,
und wobei das Gewichtsverhältnis des Gehalts des Carboxyvinylpolymers zu dem Gehalt des Wirkstoffs in dem Beschichtungsmittel 5:1 bis 1:100 beträgt.

2. Mikronadelvorrichtung gemäß Anspruch 1, wobei der Gehalt des Carboxyvinylpolymers in dem Beschichtungsmittel 0,5 bis 10 Gew.-% beträgt.

3. Mikronadelvorrichtung gemäß Anspruch 1, wobei das Gewichtsverhältnis des Gehalts des Carboxyvinylpolymers zu dem Gehalt des Wirkstoffs in dem Beschichtungsmittel 1:3 bis 1:80 beträgt.

## Revendications

1. Dispositif à micro-aiguilles comprenant :
une base ; et
des micro-aiguilles capables de percer la peau et d'être disposées sur la base,
dans lequel au moins une partie de la surface des micro-aiguilles et/ou de la base est enduite d'un agent d'enduction comprenant un ingrédient actif présentant un poids moléculaire de 1 000 ou plus et un polymère carboxyvinylique, et dans lequel l'enduction peut être obtenue par l'application d'une solution d'enduction présentant un pH de 5,2 à 5,4,
la teneur du polymère carboxyvinylique dans l'agent d'enduction étant de 0,1 à 30 % en poids,
et le rapport en poids de la teneur du polymère carboxyvinylique à la teneur de l'ingrédient actif dans l'agent d'enduction étant de 5:1 à 1:100.

2. Dispositif à micro-aiguilles selon la revendication 1, dans lequel la teneur du polymère carboxyvinylique dans l'agent d'enduction est de 0,5 à 10 % en poids.

3. Dispositif à micro-aiguilles selon la revendication 1, dans lequel le rapport en poids de la teneur du polymère carboxyvinylique à la teneur de l'ingrédient actif dans l'agent d'enduction est de 1:3 à 1:80.
